COMPLETE DOCUMENT

(19) Europäisches Patentamt
European Patent Office
Office européen des brevets

(11) Publication number: **0 086 043**
**A1**

(12) # EUROPEAN PATENT APPLICATION

(21) Application number: 83300222.3

(22) Date of filing: 18.01.83

(51) Int. Cl.³: C 07 D 233/64
A 61 K 31/415

(30) Priority: 27.01.82 GB 8202329

(43) Date of publication of application:
17.08.83 Bulletin 83/33

(84) Designated Contracting States:
AT BE CH DE FR GB IT LI LU NL SE

(71) Applicant: Pfizer Limited
Ramsgate Road
Sandwich Kent CT13 9NJ(GB)

(84) Designated Contracting States:
GB

(71) Applicant: Pfizer Corporation
Calle 15 1/2 Avenida Santa Isabel
Colon(PA)

(84) Designated Contracting States:
BE CH DE FR IT LI LU NL SE AT

(72) Inventor: Banks, Bernard Joseph
18 Victoria Avenue St. Peters
Broadstairs Kent(GB)

(72) Inventor: Penrose, Alexander Ballinghall
Omega Lower Street
Tilmanstone Nr. Deal Kent(GB)

(74) Representative: Moore, James William
Pfizer Limited Ramsgate Road
Sandwich Kent CT13 9NJ(GB)

(54) **2-(Phenoxyalkyl) imidazoles as ectoparasiticides and anthelmintics.**

(57) 2-(Phenoxyalkyl)imidazoles of the formula:

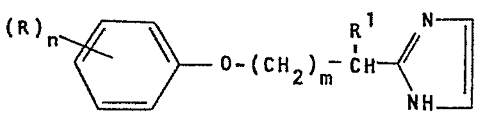

---(I)

wherein $(R)_n$ represents up to 5 optional substituents, n being 0 or an integer of from 1 to 5 and each R is independently hydrogen, halo or a $C_1$-$C_4$ alkyl, $C_1$-$C_4$ alkoxy of perfluoroalkyl group; or two adjacent R groups taken together may be $-(CH_2)_3-$ or $-(CH_2)_4-$; m is 0, 1 or 2; and $R^1$ is hydrogen or a $C_1$-$C_6$ alkyl group; and the physiologically acceptable acid addition salts thereof; are useful for combating ectoparasite (especially acaricide) and helminth infections in sheep and cattle.

EP 0 086 043 A1

This invention relates to a series of imidazole derivatives having ectoparasiticidal activity and, in particular, to certain 2-(phenoxyalkyl)imidazoles having acaricidal, insecticidal and miticidal activity.

Eggs are laid by adult female acarids on animal skins, and the larvae produced tend to burrow into the skins of the afflicted animals and thereby spoil the state of the skins, with the consequence, for example, that cattle hides and sheep skins and fleece intended for the manufacture of leather, sheepskin and woollen goods, respectively, are reduced in quality. Furthermore, the general state of health and the quality of the flesh of afflicted animals may be detrimentally affected. Certain insect larvae, for example, the larvae of blowflies which tend to live in sheep skin, are capable of bringing premature death to the animal if present in sufficient abundance.

The compounds of the invention are acaricides with good expellency properties, particularly against cattle ticks, and also cattle and sheep miticides. The compounds are also active against blowfly larvae and against the adult and larval stages of nuisance flies which can cause severe distress to cattle. Certain of the compounds also have anthelmintic activity, as shown by their activity against Caenorhabditis elegans.

European patent application no. 11596 describes certain 2($\alpha$-phenoxyalkyl)imidazoline derivatives as miticides useful against plant and animal parasites. However, the imidazoline derivatives suffer from the disadvantage that they tend to be

unstable in aqueous solution, particularly in the presence of traces of acids or bases.

The imidazole derivatives of the present invention are potent antiparasitic agents having good stability in aqueous solutions which enables them to be used more advantageously and conveniently, for example, in aqueous dips and sprays.

Certain 2-aryloxymethylimidazoles of formula I as herein defined, wherein m is 0 and $R^1$ is H, within the scope of the present invention, are disclosed in J. Heterocyclic Chemistry, 1973, 10, 391. The compounds were prepared and tested as anorectic agents and there is no suggestion that the compounds could have any anti-parasitic or insecticidal properties.

Thus, according to the present invention there are provided compounds of the general formula:

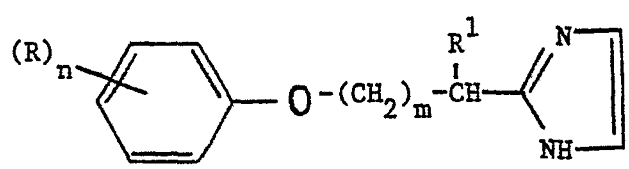

--- (I)

wherein $(R)_n$ represents up to 5 optional substituents, n being 0 or an integer of from 1 to 5 and each R is independently hydrogen, halo or a $C_1$-$C_4$ alkyl, $C_1$-$C_4$ alkoxy or perfluoroalkyl group; or two adjacent R groups taken together may be $-(CH_2)_3-$ or $-(CH_2)_4-$;

m is 0, 1 or 2;

and $R^1$ is hydrogen or a $C_1$-$C_6$ alkyl group;

and the physiologically acceptable acid addition salts thereof, with the proviso that $(R)_n$ may not be hydrogen, mono- or 2,4- or 3,4-di-chloro, 4-methoxy or 2,4-dimethyl when m is 0 and $R^1$ is hydrogen.

Halo, as used herein, means fluoro, chloro, bromo or iodo. Alkyl and alkoxy groups of 3 or more carbon atoms may be straight or branched chain. Physiologically acceptable acid addition salts include those salts which are commonly used in accordance with normal veterinary practice and which are non-irritant when applied externally to an animal and not unduly toxic if ingested in small amounts.

The invention also includes ectoparasiticidal and anthelmintic compositions, including concentrates, comprising a compound of the formula I, without proviso, or a physiologically acceptable acid addition salt thereof, together with a diluent or carrier. Thus, as well as including compositions of the novel compounds of formula I, the invention includes ectoparasiticidal and anthelmintic compositions containing a known compound of the formula I and a diluent or carrier, when the composition is in a form not disclosed in the Journal of Heterocyclic Chemistry reference i.e. when it is other than a 0.5 percent methyl cellulose solution.

The invention further includes a compound of the formula I, <u>without proviso</u>, or a physiologically acceptable acid addition salt thereof, for use in treating an animal to combat ectoparasite and helminth infestations.

The preferred substituent groups R are halogen or lower alkyl, particularly where $(R)_n$ is 2,3-dimethyl.

$R^1$ is preferably hydrogen or methyl and m is preferably 0.

Particularly preferred individual compounds include 2-(2,3-dimethylphenoxymethyl)imidazole and 2-[1-(2,3-dimethylphenoxy)ethyl]imidazole.

The compounds of the formula I are prepared according to the following reaction scheme wherein Z is CN or $CONH_2$; R, $R^1$, n and m are as previously defined and $R^2$ and $R^3$ are $C_1$-$C_4$ alkyl groups.

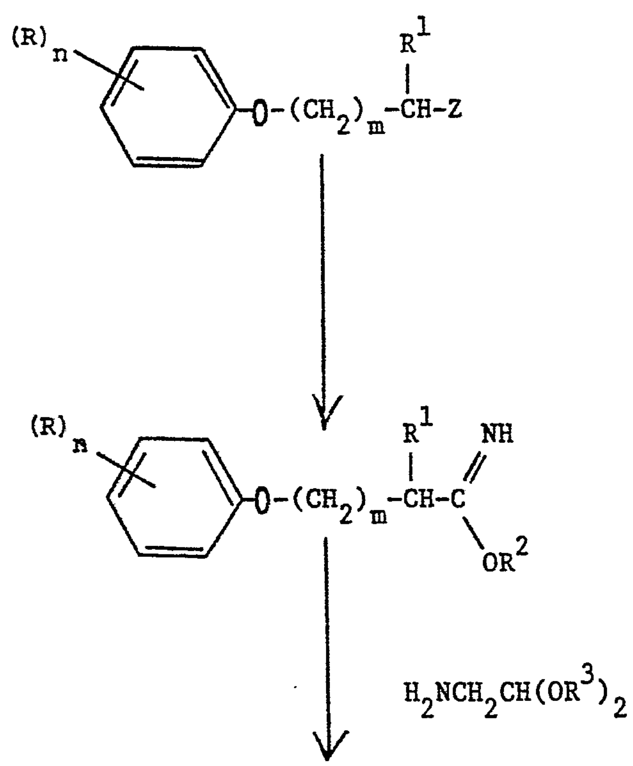


--- (II)

--- (III)

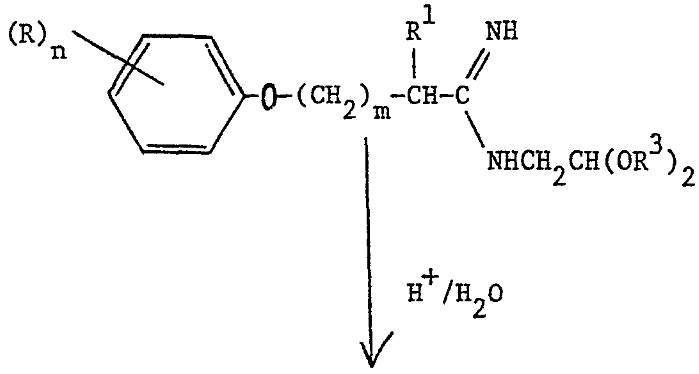


--- (IV)

(I)

In the case of the nitriles of formula (II) wherein Z in CN, the conversion to the intermediate imidate ester (III) is achieved by reaction with gaseous hydrogen chloride and a $C_1$-$C_4$ lower alkanol of formula $R^2OH$ (e.g. ethanol), in a reaction-inert organic solvent e.g. dichloromethane. In a typical procedure the nitrile and alkanol in equimolar amounts are dissolved in dichloromethane and the solution cooled and saturated with hydrogen chloride gas. After several hours at 0°C the solvent is evaporated and the intermediate imidate ester (III) isolated as its hydrochloride salt.

When starting from an amide of formula (II) wherein Z is $CONH_2$, the conversion to the imidate ester is achieved using a trialkyloxonium tetrafluoroborate, e.g. trimethyloxonium tetrafluoroborate (Meerwein's reagent). The reagents, in equimolar amounts, are generally added to a reaction inert organic

solvent, e.g. dichloromethane, and allowed to react at room temperature. A period of 24 hours is generally sufficient, at which time the solvent is removed to give the imidate ester III as its tetrafluoroborate salt.

The method starting with a nitrile of formula II wherein Z is CN is generally preferable and is especially applicable to those compounds wherein $R^1$ is hydrogen or methyl. Compounds wherein $R^1$ is a $C_2$ to $C_6$ alkyl group are best prepared via the amides of formula II wherein Z is $CONH_2$. In either case the resulting intermediate imidate ester of formula (III) is reacted directly with an aminoacetaldehyde di-lower alkyl acetal of formula $H_2NCH_2CH(OR^3)_2$ where $R^3$ is a $C_1$-$C_4$ alkyl group, to give the intermediate (IV). The reaction is conveniently performed in a lower alkanol solvent, the groups $R^2$, $R^3$ and the alkyl content of the solvent ideally being the same. Thus the reaction may be achieved using aminoacetaldehyde diethylacetal in ethanol. The acetal and the imidate ester are generally used in equimolar amounts and the reaction is usually substantially complete within a period of 48 hours at room temperature.

The intermediate (IV) is not usually isolated, the cyclisation step to give the imidazole of formula (I) being readily achieved by the addition of aqueous acid. Thus after removal of the solvent the crude compound of formula (IV) is treated with hot aqueous acid, e.g. hydrochloric acid, for a period of 30 minutes or so at 90-100°C. The product is finally

worked up in a conventional manner, e.g. by neutralising the acidic solution, and isolating the crude product by filtration or, if soluble, by solvent extraction. Further purification can be performed, if desired, for example by crystallization or by chromatography. The product may either be isolated as the free base or, by adding an appropriate acid, as an acid addition salt.

The starting materials of formula (II) are generally known compounds which may be prepared by conventional methods. For example the nitriles of formula II may be prepared from a phenol by reaction with a α-chloroalkylnitrile according to the method of Djerassi and Scholz, J. Amer. Chem. Soc., 1947, 69, 1690. The amides are similarly prepared by conventional procedures, for example by reaction of a phenol with an ethyl α-bromoalkanoate followed by conversion of the ester to the amide by a conventional reaction with concentrated ammonia solution.

Compounds where $R^1$ is other than hydrogen can exist in optically active isomeric forms and the invention includes the separated isomers as well as racemic mixtures thereof.

The antiparasitic and insecticidal properties of the compounds of the invention are measured by the following tests:

In one test, to assess acaricidal activity, five freshly collected, fully engorged *Boophilus microplus* female ticks are used for each acaricidal compound. Using a micro-pipette 10 micro-litres of a solution containing 10 micro-grams of the acaricidal compound in ethanol or acetone, is applied to the dorsal surface of each of the ticks. The treated ticks are placed

in weighed 1" x 2" glass vials, weighed and stored at 26°C and 80% relative humidity in plastic boxes for two weeks. The ticks are then removed from the vials and the vials weighed to give the weight of eggs laid by the ticks. Any reduction in the egg laying of the treated ticks is calculated as a percentage of the eggs laid by untreated control ticks.

The eggs are returned to the incubator for a further 3 weeks after which time the percentage of eggs hatching is estimated. The percentage effect is calculated as the overall reduction in the anticipated reproduction of the ticks using the weight of eggs laid and the percentage of eggs hatching. The test may be repeated using smaller amounts of the acaricidal compound.

In another test, using a pipette 0.5 ml of a solution containing 0.5 mg of the acaricidal compound in ethanol or acetone is spread evenly on to a filter paper 8 cm x 6.25 cm (50 sq. c.m.) to give a dosage of 100 $mg/m^2$. The treated paper is allowed to dry at room temperature, folded with the treated surface inside and two short edges sealed with a crimping machine. The open ended envelope is placed in a 1lb Kilner jar containing damp cotton wool in a plastic pot and stored in an incubator at 26°C for 24 hours. 20-50 Boophilus microplus larvae, which had hatched 8-14 days previously, are placed in the envelope using a small spatula. The open end is then crimped to form a sealed packet.

The treated paper containing the larvae is returned to the Kilner jar and kept for a further 48 hours in the incubator. 20-50 larvae are placed similarly in an untreated paper envelope to act as controls. At the end of the 48 hour test period the mortality is noted and recorded as a percentage after correction for any mortality among the untreated control ticks.

The test may be repeated using smaller amounts of the acaricidal compound. Activity against Haemaphysalis longicornus nymphs may be measured in a similar manner to the above larvae test.

Larvicidal properties are investigated by maintaining test and control groups of Lucilia cuprina (blowfly) larvae in separate test tubes, each containing filter paper partially soaked in calf serum serving as food and plugged with cotton wool. The filter paper in the test tube containing the test larvae is additionally impregnated with the compound under investigation to the extent of a 100 $mg/m^2$ deposition. Both test tubes are stored with the top part only in a strong light so as to induce the larvae to stay in the lower part of the tubes in contact with the filter paper through exploitation of their aversion to light. Mortality is noted and recorded as a corrected percentage. The finding of substantial numbers of test larvae on the illuminated plug suggests that the test compound has marked repellant properties.

In addition to percentage effectiveness figures, $LD_{90}$ results can be obtained from dose response measurements using any of the afore-described tests.

In anthelmintic testing, the compounds are screened against Caenorhabditis elegans worms in vitro. The assay is based upon the published work of Simpkin and Coles, J. Chem. Tech. and Biotechnol. 31, 66-69, (1981).

In mite testing, two species of mites are used. These are Dermanyssus gallinae (Acarina, Mesostigmata), the poultry red mite, and Psoroptes cuniculi (Acarina, Astigmata), the rabbit ear mite. The test procedure, using 30-50 mites, is identical to that described for Boophilus microplus larvae above.

The compositions of the invention include not only compositions in a suitable form for application but concentrated primary compositions which may be supplied to the user and which require dilution with a suitable quantity of water or other diluent prior to application. Typical compositions of the invention for use in treating ectoparasitic infections of animals such as sheep and cattle include, for example, dusting powders, dispersible powders, dips, sprays, dispersions, emulsions and emulsifiable concentrates.

A dust may be made by mixing the appropriate amount of the finely divided active compound with a solid pulverulent diluent or carrier such as talc, clay, calcite, pyrophyllite, diatomaceous earth, walnut shell flour, silica gel, hydrated alumina, or calcium silicate. As an alternative method of preparation, the diluent or carrier is mixed with a solution of the active compound in a volatile organic solvent such as toluene, the solvent being subsequently removed by evaporation. Typically, the active

compound will be present in the dust in an amount of from 0.25 to about 4% by weight.

Dispersible powders of special value for spray applications may be made by adding a suitable wetting and dispersing agent to the active compound, or to a dust containing the active compound, so that a stable aqueous dispersion of the active compound is formed on mixing the powder with water. The dispersible powders preferably contain from about 25 to 75% by weight of the active ingredient.

Emulsifiable concentrates comprise a solution of the active compound in a substantially water-immiscible non-toxic organic solvent containing an emulsifying agent. Suitable solvents include, for example, toluene, xylene, petroleum oil, and alkylated naphthalenes. Suitable emulsifiers, which can be cationic, anionic or non-ionic are well known to those skilled in the art, and include ordinary soaps (anionic), lauryl pyridinium chloride (cationic) and polyoxethylene lauryl ethers (non-ionic). Preferably, the concentrate will contain 5-75 gms. of the active compound per 100 ml. of solution. The concentrates may be diluted with water prior to use to give a typical concentration of the active compound in the aqueous medium of from about 0.01 to about 0.1 g/100 ml. or approximately 100 to 1000 p.p.m. The volatile solvents evaporate after application to the animal to leave a deposit of the active ingredient.

For treatment of helminth infections the compounds are most readily administered by incorporation into animal feeds, although

administration in the form of a prolonged release bolus, implant or injection is also possible. For the control of nuisance flies breeding in dung, the compounds may also be incorporated into animal feeds. Thus the invention includes an animal feed or concentrate therefor including a compound of the formula I as hereinbefore defined without proviso.

The compositions of the invention may also contain a pesticide, fungicide, additional acaricide or the like and they may be administered alone or in combination with conventional ectoparasiticidal agents such as organophosphates, carbamates, organochlorines, pyrethroids, formamidines, triazapentadienes, triazinethiones or thioureas.

The invention is further illustrated by the following Examples: Nuclear magnetic resonance spectral date (n.m.r.) where given were measured at 60 $MH_z$ for solutions in deuterochloroform ($CDCl_3$) and peak positions are given in p.p.m. downfield from tetramethylsilane with the following abreviations for peak shapes: b, broad; s, singlet; d, doublet; t, triplet; q, quarter and m, multiplet.

## EXAMPLE 1

### 2-(2,3-Dimethylphenoxymethyl)imidazole

2,3-Dimethylphenoxyacetonitrile (150 g, 0.93 mole) was dissolved in a mixture of dry dichloromethane (1000 ml) and dry ethanol (42.8 g, 0.93 mole). The solution was cooled to -10°C and saturated with hydrogen chloride gas. After stirring at 0°C for

two hours the dichloromethane was removed under reduced pressure and dry diethyl ether (100 ml) added. The resulting slurry was filtered and the residue washed with dry diethyl ether (500 ml) and dried under vacuum for one hour to give the intermediate imidate ester hydrochloride (212 g) which was used without further purification.

The imidate ester hydrochloride (150 g, 0.618 mole) was dissolved in absolute ethanol (2000 ml) at room temperature and aminoacetaldehyde diethylacetal (82.4 g, 0.618 mole) added. The mixture was stirred overnight at room temperature and the solvent removed under reduced pressure. The residue was taken up with hot 6N hydrochloric acid (200 ml) and the solution maintained at 90°C for thirty minutes. The resulting mixture was diluted with water (800 ml) and basified with aqueous sodium hydroxide solution (60 g in 150 ml). The resulting precipitate was stirred for 30 minutes to facilitate granulation and then collected by filtration. The residue was dissolved in a mixture of dichloromethane (2000 ml) and methanol (200 ml). The aqueous layer was separated and the solution filtered to remove insolubles. The filtrate was stirred at reflux temperature with charcoal and magnesium sulphate, and the solution then filtered and the solvent evaporated to give the crude product (112 g). The crude product was dissolved in ethyl acetate (1000 ml) at reflux, the volume was then reduced to 400 ml and mixture set aside to crystallize at 0-10°C. The crystalline precipitate was collected by filtration and dried under vacuum to give the title product (71 g, 56%) m.p. 144-145°C.

Analysis %:-

| | |
|---|---|
| Found: | C,70.44; H,7.02; N,13.66. |
| $C_{12}H_{14}N_2O$ Requires: | C,71.26; H,6.98; N,13.85. |

EXAMPLES 2-15

The following phenoxyalkylimidazoles of formula I were prepared by the general method of Example 1, starting with the appropriate nitrile:

| Example No. | $(R)_n$ | m | $R^1$ | m.p.(°C) | Analysis % (or n.m.r.) (theoretical in brackets) C | H | N |
|---|---|---|---|---|---|---|---|
| 2 | H | 0 | H | 103-105* | 5.55 (s, 2H), 6.5-7.7 (m with s at * 7.5, 9H) | | |
| 3 | 4-Cl | 0 | H | 112-113 | 57.14<br>(57.51 | 4.33<br>4.30 | 13.19<br>13.42) |
| 4 | $4-OCH_3$ | 0 | H | 105-109 | 64.35<br>(64.69 | 5.95<br>5.92 | 13.66<br>13.72) |
| 5 | $2-CH_3$ | 0 | H | 82-84 | 70.84<br>(71.26 | 6.98<br>6.98 | 13.68<br>13.85) |
| 6 | $2,4-CH_3$ | 0 | H | 121-122 | 2.2 (s, 3H), 2.25 (s, 3H), 5.1 (s, 2H), 6.6-7.3 (m with s at 7, 5H), 7.7 (s (broad), 1H) | | |
| 7 | $2,5-CH_3$ | 0 | H | 136-138 | 71.11<br>(71.26 | 6.97<br>6.98 | 13.58<br>13.85) |
| 8 | $2,6-CH_3$ | 0 | H | 140-142 | 71.00<br>(71.26 | 7.07<br>6.98 | 13.94<br>13.85) |
| 9 | 2,3-Cl | 0 | H | 138-139 | 49.06<br>(49.40 | 3.41<br>3.32 | 11.73<br>11.52) |

| Example No. | $(R)_n$ | m | $R^1$ | m.p. (°C) | Analysis % (or n.m.r.) (theoretical in brackets) C | H | N |
|---|---|---|---|---|---|---|---|
| 10 | $2,3-(CH_2)_4-$ | 0 | H | 148-151 | 73.13 (73.65 | 7.20 7.06 | 11.95 12.27) |
| 11 | $2,3-CH_3$ | 1 | H | 100-101 | m/e 217 (M+1), 216 (M), 95 ($M-C_8H_9O$) | | |
| 12 | $2,3-CH_3$ | 2 | H | 109-110 | 2.0-2.5 (m with s at 2.1 and s at 2.25, 8H), 2.95 (t, J=6Hz, 2H), 3.95 (t, J=6Hz, 2H), 6.5-7.3 (m with s at 6.9, 5H), 8.6 (s (broad), 1H) | | |
| 13 | $2,3-CH_3$ | 0 | $CH_3$ | 171-173* | 61.73 (61.78 | 6.84 6.78 | 10.99 * 11.08 |
| 14 | $2,3,6-CH_3$ | 0 | $CH_3$ | 180-182 | 72.72 (73.01 | 7.90 7.88 | 12.15 12.70) |
| 15 | $3,5-CH_3$ | 0 | H | 134-135 | 70.88 (71.26 | 6.92 6.98 | 13.98 13.85) |

* Hydrochloride Salt

0086043

## EXAMPLE 16

### 2-[1-(2,3-Dimethylphenoxy)propyl]imidazole

Trimethyloxonium tetrafluoroborate (3.1 g, 0.21 mole) was added to a solution of 1-(2,3-dimethylphenoxy)butyramide (4.14 g, 0.02 mole) in anhydrous dichloromethane (250 ml), and the mixture was stirred at room temperature under an atmosphere of dry nitrogen for 24 hours. The dichloromethane was removed under vacuum and the residue taken up in anhydrous ethanol (100 ml). Aminoacetaldehyde diethyl acetal (2.66 g, 0.02 mole) was added and the mixture was stirred at room temperature under an atmosphere of dry nitrogen for 48 hours. The solution was concentrated under vacuum to a volume of approximately 15 mls, added to aqueous hydrochloric acid (100 ml, 5N) and the solution maintained at around 90°C on a steam bath for 30 minutes. The cooled solution was washed with diethyl ether (3 x 30 ml), and the aqueous layer was then adjusted to pH12 with sodium hydroxide solution and extracted with ethyl acetate (3 x 30 ml). The combined ethyl acetate extracts were dried ($MgSO_4$) and evaporated. The resulting oil was taken up in chloroform (10 ml) and chromatographed on silicic acid (50 g) eluting with a 1% solution of methanol in chloroform. Combination of the relevant fractions and evaporation of the solvent gave the product as a gum, (104 mg, 2.2%).

<u>N.M.R.</u> ($CDCl_3$): 1.02 (t, J=7Hz, 3H), 2.05 (q, J=7Hz, 2H), 2.24 (s, 3H), 2.29 (s, 3H), 5.2-5.41 (m, 1H), 6.5-7.2 (m, 3H), 7.29 (s, 2H).

0086043

## EXAMPLE 17

### 2-[1-(2,3-Dimethylphenoxy)butyl]imidazole

The procedure of Example 16 was followed but starting with 1-(2,3-dimethylphenoxy)propionamide to give the title compound as a gum.

N.M.R.: ($CDCl_3$): 0.95 (t, J=7Hz, 3H); 1.15-1.65 (m,2H), 1.9-2.06 (m,2H), 2.1 (s,3H), 2.15 (s,3H), 5.4 (t,J=7Hz,1H), 6.6-7.2 (m with s at 7.02,5H).

## EXAMPLE 18

An emulsifiable concentrate containing the compound of Example 1 is prepared by the thorough mixing of the following ingredients:

| Constituent | % w/w |
|---|---|
| Product of Example 1 | 12.5 |
| "Arylan CA" (Trade mark, p-dodecylbenzene-sulfonate, anionic surfactant) | 4.0 |
| "Ethylan BV" (Trade mark, non-ionic surfactant) | 12.0 |
| "Solvesso 200" (Trade mark, mixture of methyl napthalenes) | 71.5 |

The concentrate is mixed with water to provide an animal dip bath of the required concentration.

CLAIMS FOR BE, CH, DE, FR, GB, IT, LI, LU, NL and SE

1. A compound of the general formula:

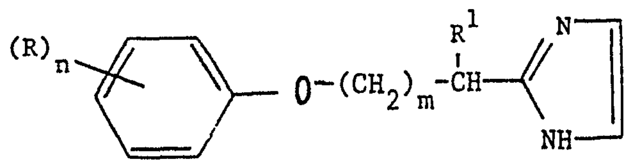 --- (I)

wherein $(R)_n$ represents up to 5 optional substituents, n being 0 or an integer of from 1 to 5 and each R is independently hydrogen, halo or a $C_1$-$C_4$ alkyl, $C_1$-$C_4$ alkoxy or perfluoroalkyl group; or two adjacent R groups taken together may be $-(CH_2)_3-$ or $-(CH_2)_4-$;

m is 0, 1 or 2;

and $R^1$ is hydrogen or a $C_1$-$C_6$ alkyl group;

and the physiologically acceptable acid addition salts thereof, with the proviso that $(R)_n$ may not be hydrogen, mono- or 2,4- or 3,4-di-chloro, 4-methoxy or 2,4-dimethyl when X is oxygen, m is 0 and $R^1$ is hydrogen.

2. A compound as claimed in claim 1 wherein R is halo or $C_1$-$C_4$ alkyl.

3. A compound as claimed in claim 2 wherein $(R)_n$ is 2,3-dimethyl.

4. A compound as claimed in any one of claims 1 to 4 wherein $R^1$ is hydrogen or methyl and m is 0.

5. A compound as claimed in claim 5 wherein said compound is 2-(2,3-dimethylphenoxymethyl)imidazole or 2-[1-(2,3-dimethylphenoxy)ethyl]imidazole.

6. An ectoparasiticidal, especially acaricidal, or anthelmintic composition, which comprises a compound of the formula (I) as claimed in claim 1 without proviso or in any one of claims 2 to 5, or a physiologically acceptable acid addition salt thereof, together with a diluent or carrier.

7. A composition as claimed in claim 6, which is in the form of an emulsifiable concentrate, dust, dispersible powder, injectable formulation, prolonged-release bolus, or aqueous emulsion.

8. A compound of the formula (I) or a physiologically acceptable acid addition salt thereof as claimed in claim 1 without proviso or in any one of claims 2 to 5, or a composition thereof as claimed in claim 6 or claim 7 for use in treating an animal, especially sheep or cattle, to combat ectoparasite, especially acaricide, and helminth infestations.

9. An animal feed containing a compound of the formula I, or a physiologically acceptable acid addition salt thereof, as claimed in claim 1 without proviso or in any one of claims 2 to 5.

10. A process for preparing a compound of the formula (I) as claimed in claim 1 (with proviso) characterised by reacting a compound of the formula:

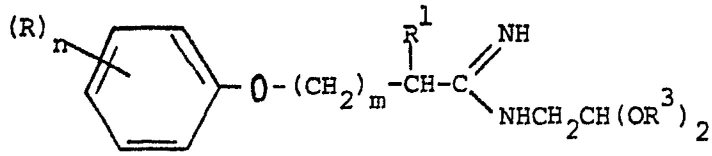

--- (IV)

wherein R, $R^1$, n and m are as defined in claim 1 and $R^3$ is a $C_1$-$C_4$ alkyl group;

with aqueous acid and isolating the compound of formula (I) and, if desired, preparing a pysiologically acceptable acid addition salt thereof.

11. A method of combatting ectoparasite infestations in an animal, especially a sheep or cattle, which comprises applying an effective amount of a compound of the formula (I), or a physiologically acceptable acid addition salt thereof, as claimed in claim 1, without proviso, or in any one of claims 2 to 5, to the exterior of the animal.

CLAIMS FOR AUSTRIA

1. A process for preparing an ectoparasiticidal, especially acaricidal, or anthelmintic composition, characterised by mixing a compound of the formula:-

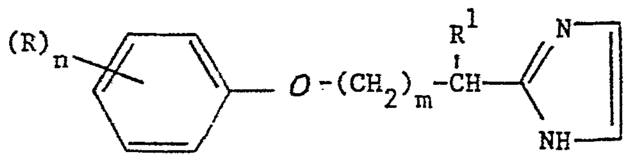

--- (I)

wherein $(R)_n$ represents up to 5 optional substituents, n being 0 or an integer of from 1 to 5 and each R is independently hydrogen, halo or a $C_1$-$C_4$ alkyl, $C_1$-$C_4$ alkoxy or perfluoroalkyl group; or two adjacent R groups taken together may be $-(CH_2)_3-$ or $-(CH_2)_4-$;

m is 0, 1 or 2;

and $R^1$ is hydrogen or a $C_1$-$C_6$ alkyl group;

or a physiologically acceptable acid addition salt thereof; with a diluent or carrier.

2. A process for preparing a compound of the formula I as defined in claim 1 or a physiologically acceptable acid addition salt thereof, with the proviso that $(R)_n$ may not be hydrogen, mono- or 2,4- or 3,4-di-chloro, 4-methoxy or 2,4-dimethyl when m is 0 and $R^1$ is hydrogen, characterised by reacting a compound of the formula:

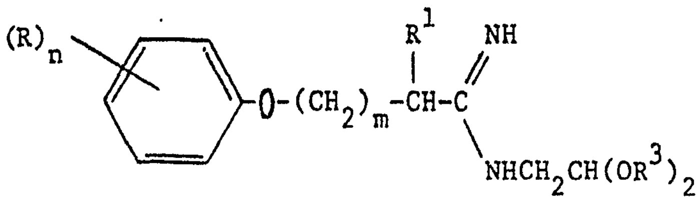

--- (IV)

wherein R, $R^1$, n and m are as defined in claim 1 and $R^3$ is a $C_1$-$C_4$ alkyl group, with aqueous acid and isolating the compound of formula (I) and, if desired preparing a physiologically acceptable acid addition salt thereof.

3. A process as claimed in claim 2 wherein the reaction is performed using aqueous hydrochloric acid at 90-100°C.

4. A process according to claim 2 or claim 3 characterised in that R is halo or $C_1$-$C_4$ alkyl.

5. A process according to claim 4 characterised in that $(R)_n$ is 2,3-dimethyl, $R^1$ is hydrogen or methyl and m is 0.

European Patent Office

# EUROPEAN SEARCH REPORT

0086043
Application number

EP 83 30 0222

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| X | US-A-4 031 232 (WINKELMANN-RAETHER) *Columns 3-4,14,(ex.110)* --- | 1,4 | C 07 D 233/64<br>A 61 K 31/415 |
| X | JOURNAL OF MEDICINAL CHEMISTRY, vol. 13, no. 5, September 1970, pages 968-970, Washington D.C. (US); L.R.Swett et al.: "Imidazole derivatives.Histidine decarboxylase inhibitors" *Page 970* --- | 6,8 | |
| A | JOURNAL OF MEDICINAL CHEMISTRY. *Page 969, (no.19)* --- | 1-2,4 | |
| D | JOURNAL OF HETEROCYCLIC CHEMISTRY, vol.10, no. 3, June 1973, pages 391-4, Provo Utah (US); E.R.Freiter et al.: "Synthesis of a series of 2-arcyloxymethylimidazoles" *Pages 392-3* ----- | 6-8 | TECHNICAL FIELDS SEARCHED (Int. Cl. 3)<br>C 07 D 233/00<br>A 61 K 31/00 |

The present search report has been drawn up for all claims

| Place of search | Date of completion of the search | Examiner |
|---|---|---|
| THE HAGUE | 22-04-1983 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82